# FASCICULE DE BREVET EUROPEEN

(11) **EP 2 464 742 B1**
(45) Date de publication et mention de la délivrance du brevet: **04.10.2017**
(21) Numéro de dépôt: 10761051.1
(22) Date de dépôt: 12.08.2010
(51) Int. Cl.: C12Q 1/14

(54) **MILIEU REACTIONNEL POUR LES BACTERIES STAPHYLOCOCCUS AUREUS RESISTANTES A LA METICILLINE (MRSA)**
REAKTIONSMEDIUM FÜR METHICILLIN-RESISTENTE STAPHYLOCOCCUS AUREUS (MRSA)-BAKTERIEN
REACTION MEDIUM FOR METHICILLIN-RESISTANT STAPHYLOCOCCUS AUREUS (MRSA) BACTERIA

(30) Priorité: 13.08.2009 FR 0903944
(43) Date de publication de la demande: 20.06.2012
(73) Titulaire: Biomérieux, 69280 Marcy L'Etoile (FR)
(72) Inventeur: ZAMBARDI, Gilles, F-38300 Chezeneuve (FR); ROCHE, Jean-Marc, F-73350 Feissons Sur Salins (FR)
(86) Numéro de dépôt international: PCT/FR2010/051705
(87) Numéro de publication internationale: WO 2011/018589

(56) Documents cités:
- EP-A2- 0 887 424
- WO-A1-2004/027086
- WO-A2-02/079486
- DATABASE WPI Week 199511 Thomson Scientific, London, GB; AN 1995-077141 XP002573758, & JP 7 000181 A (KYOKUTO SEIYAKU KOGYO KK) 6 janvier 1995 (1995-01-06)
- MANAFI M ET AL: "FLUOROGENIC AND CHROMOGENIC SUBSTRATES USED IN BACTERIAL DIAGNOSTICS", MICROBIOLOGICAL REVIEWS, AMERICAN SOCIETY FOR MICROBIOLOGY, WASHINGTON, DC, US, vol. +, no. 3, 1 septembre 1991 (1991-09-01), pages 335-348, XP009020939, ISSN: 0146-0749
- ENGELS W ET AL: "Rapid and direct staphylocoagulase assay that uses a chromogenic substrate for identification of Staphylococcus aureus.", JOURNAL OF CLINICAL MICROBIOLOGY NOV 1981, vol. 14, no. 5, November 1981 (1981-11), pages 496-500, ISSN: 0095-1137
- LANGLET S ET AL: "[A chromogenic method for rapid identification of Staphylococcus aureus].", ANNALES DE BIOLOGIE CLINIQUE 1999 MAR-APR, vol. 57, no. 2, March 1999 (1999-03), pages 191-196, ISSN: 0003-3898
- Piyarak Tanprasert ET AL: "Determination of minimal bactericidal and effective antibiotic treatment concentrations for bacterial contaminants from micropropagated strawberries", In Vitro Cellular & Developmental Biology - Plant, vol. 33, no. 3, 1 July 1997 (1997-07-01), pages 227-230, XP055214889, ISSN: 1054-5476, DOI: 10.1007/s11627-997-0027-5

## Description

La présente invention concerne un milieu réactionnel pour la détection des bactéries *Staphylococcus aureus* résistantes à la Méticilline (SARM ou MRSA). L'invention concerne également l'utilisation de ce milieu, ainsi qu'un procédé pour identifier des bactéries MRSA.

Les *Staphylococcus aureus* résistants à la Méticilline (MRSA) sont des souches de *Staphylococcus aureus,* caractérisées par leur résistance à un antibiotique, la Méticilline, et aux antibiotiques apparentés telle que l'Oxacilline. Les bactéries MRSA représentent un fort pourcentage des infections nosocomiales, et sont souvent à l'origine de problèmes de santé graves et potentiellement mortels. Transmis le plus souvent de façon croisée entre les patients via le personnel soignant, les MRSA sont responsables d'infections endémiques très difficiles à contrôler. Outre un traitement adapté, le dépistage des porteurs de MRSA, et l'isolement des patients colonisés constituent la méthode la plus efficace, aujourd'hui recommandée par des organismes officiels tels que la Society for Healthcare Epidemiology of America (Société Américaine pour l'Epidémiologie Hospitalière). Un dépistage précoce et systématique est donc essentiel.

La détection des MRSA peut se faire par différentes techniques.

Il est ainsi possible de détecter des MRSA par des techniques de biologie moléculaire. A ce titre, on peut citer notamment la demande EP887424. De telles méthodes restent toutefois coûteuses en test de routine, et requierent un personnel qualifié.

Il est également possible d'utiliser des milieux de culture conventionnels pour détecter les *Staphylococcus aureus,* tel que le milieu décrit dans la demande EP1390524. La détection de MRSA se fait lors d'une étape supplémentaire, par un test d'agglutination spécifique, (Slidex MRSA, bioMérieux) ou par une méthode de diffusion sur gélose en présence d'un disque Oxacilline de Céfoxitine ou de Latamoxef (recommandations du Comité de l'Antibiogramme de la Société Française de Microbiologie et du Clinical Laboratory Standard Institute).

Il est également possible de mettre en culture les bactéries susceptibles d'être des MRSA sur milieux gélosés en présence d'antibiotiques. De tels milieux peuvent être également chromogènes, ce qui facilite la lecture et la détection des MRSA. On peut citer notamment le milieu décrit dans la demande EP1543147. Toutefois, la détection d'une activité phosphatase dans les conditions décrites étant peu spécifique, il est nécessaire de la combiner avec la détection de plusieurs autres activités enzymatiques ce qui réduit la fertilité du milieu et accroît son coût. De même, on peut aussi citer WO 2004/027086 qui divulgue une méthode et un milieu réactionnel pour isoler des MRSA. Ledit milieu réactionnel est un milieu comprenant un substrat chromogénique et, à titre d'agent sélectif, un antibiotique choisi dans le groupe des céphalosporines de seconde ou de troisième génération. Là encore, une sélectivité réduite est à déplorer. En particulier, la présence de staphylocoques. à coagulase négative (SNC) poussant sur un tel milieu réactionnel a pu être constatée.

L'invention, telle que définie par la revendication 1, se propose de résoudre les lacunes de l'état de la technique en présentant un nouveau milieu réactionnel, sensible, spécifique, et permettant un isolement et une identification rapide des *Staphylococcus aureus* résistants à la Méticilline (MRSA).
D'une manière surprenante, les inventeurs ont mis en évidence que l'utilisation d'une combinaison particulière d'antibiotiques, comprenant une céphalosporine et un aminoside, permettait d'obtenir un excellent milieu réactionnel pour isoler et identifier des *Staphylococcus aureus* résistants à la Méticilline (MRSA). En l'occurrence, ledit aminoside est la Kanamycine à une concentration comprise entre 0,2 et 1,5 mg/l ou l'Amikacine à une concentration comprise entre 0,5 et 5 mg/l.

Avant d'aller plus avant, les définitions suivantes, nullement limitatives, permettront de mieux comprendre l'invention.
Au sens de la présente invention, on entend par milieu réactionnel, un milieu comprenant tous les éléments nécessaires à la survie et/ou à la croissance de microorganismes, telles que les *Staphylococcus aureus.*
Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture.
Le milieu réactionnel peut être solide, semi-solide ou liquide. Par milieu solide, on entend par exemple un milieu gélifié. Préférentiellement, le milieu selon l'invention est un milieu gélifié. L'agar est l'agent gélifiant traditionnel en microbiologie pour la culture des microorganismes, mais il est possible d'utiliser d'autres agents gélifiants comme par exemple la gelrite, la gélatine ou l'agarose. Un certain nombre de préparations sont disponibles dans le commerce, comme par exemple l'agar Columbia, la gélose Trypcase-soja, la gélose Mac Conkey, la gélose Chapman, la gélose Sabouraud ou plus généralement celles décrites dans le Handbook of Microbiological Media (CRC Press).

Le milieu réactionnel selon l'invention peut contenir d'éventuels autres additifs comme par exemple : des peptones, un ou plusieurs facteurs de croissance, des hydrates de carbone, un ou plusieurs agents sélectifs, des solutions tampons, un ou plusieurs gélifiants... Ce milieu réactionnel peut se présenter sous forme de liquide, de gel prêt à l'emploi, c'est à dire prêt à l'ensemencement en tube, flacon, ou sur boite de Pétri. Lorsque la présentation est sous forme de gel en flacon, on réalise préférentiellement une régénération (passage à 100°C) préalable du milieu avant de couler en boîte de Petri.

Préférentiellement, le milieu selon l'invention est un milieu sélectif, c'est à dire un milieu comprenant des inhibiteurs qui privilégient la croissance des bactéries *Staphylococcus aureus.* On peut citer notamment le Chlorure de Lithium (LiCI), Azide de sodium (NaN3), Colistine, Amphotéricine, Aztréonam, Colimicine, Chlorure de Sodium (NaCl), Déféroxamine, composé vibriostatique O/129.

Au sens de la présente invention, le substrat d'une activité enzymatique ou métabolique est choisi parmi tout substrat pouvant être hydrolysé en un produit qui permet la détection, directe ou indirecte d'une activité enzymatique ou d'un métabolisme, telle que notamment une activité osidase, préférentiellement une activité glucuronidase, glucosidase ou galactosidase.

Il peut s'agir d'un substrat naturel ou synthétique. Le métabolisme du substrat provoque une variation des propriétés physico-chimiques du milieu réactionnel ou des cellules d'organismes. Cette variation peut être détectée par des méthodes physico-chimiques, notamment des méthodes optiques par l'oeil de l'opérateur ou à l'aide d'instruments, spectrométriques, électriques, magnétiques, ... Préférentiellement, il s'agit d'une variation des propriétés optiques, telles qu'une modification d'absorption, de fluorescence ou de luminescence.

Dans la présente demande, le terme coloration est employé pour couvrir une coloration, absorption de lumière dans le spectre visible, ou une fluorescence, absorption à une longueur d'onde (λex) et émission à une longueur d'onde supérieure (λem, λem > λex).

Au sens de la présente invention, on entend par substrat chromogène, tout substrat pouvant être hydrolysé en un produit qui permet la détection, directe ou indirecte d'une activité enzymatique, telle que notamment une activité osidase, préférentiellement une activité alpha glucosidase, estérase, préférentiellement une activité phosphatase, peptidase, préférentiellement une activité coagulase.

On peut citer notamment les substrats à base d'indoxyl, flavone, alizarine, acridine, phénoxazine, nitrophénol, nitroaniline, naphtol, catéchol, hydroxyquinoline, coumarine, hydroxyphenyl-quinoxazol-4-one. Préférentiellement, le(s) substrat(s) utilisé(s) dans la présente invention est(sont) à base d'indoxyl.

Comme substrat d'alpha glucosidase, on peut citer plus particulièrement les substrats 5-Bromo-6-chloro-3-indoxyl-alpha-glucoside; Dihydroxyflavone-alpha-glucoside ; 3,4-Cyclohexénoesculétine-alpha-glucoside ; 8-Hydroxiquinoline-alpha-glucoside ; 5-Bromo-4-chloro-3-indoxyl-alpha-glucoside ; 5-Bromo-4-chloro-3-indoxyl-N-méthyl-alpha-glucoside ; 6-Chloro-3-indoxyl-alpha-glucoside ; 5-Bromo-3-indoxyl-alpha-glucoside ; 5-Iodo-3-indoxyl-alpha-glucoside ; 6-Fluoro-3-indoxyl-alpha-glucoside ; Alizarine-alpha-glucoside ; Nitrophényl-alpha-glucoside ; 4-Méthylumbelliferyl-alpha-glucoside ; Naphtholbenzein-alpha-glucoside ; Indoxyl-N-méthyl-alpha-glucoside ; Naphtyl-alpha-glucoside ; Aminophényl-alpha-glucoside ; Dichloroaminophényl-alpha-glucoside. Préférentiellement, le substrat d'alpha glucosidase utilisé est le 5-Bromo-4-chloro-3-indoxyl-N-méthyl-alpha-glucoside ou le 5-Bromo-4-chloro-3-indoxyl-alpha-glucoside.

Comme substrat de phosphatase, on peut citer plus particulièrement les substrats 5-Bromo-6-chloro-3-indoxyl-phosphate ; 3,4-Cyclohexénoesculétine-phosphate ; 5-Bromo-4-chloro-3-indoxyl-phosphate ; 5-Bromo-4-chloro-3-indoxyl-N-méthyl-phosphate ; 6-Chloro-3-indoxyl-phosphate ; 5-Bromo-3-indoxyl-phosphate ; 5-Iodo-3-indoxyl-phosphate ; 6-Fluoro-3-indoxyl-phosphate ; Nitrophényl-phosphate ; 4-Méthylumbelliferyl-phosphate ; Indoxyl-N-méthyl-phosphate ; Naphtyl-phosphate, ELF97-phosphate. Préférentiellement, le substrat de phosphatase utilisé est le 6-Chloro-3-indoxyl-phosphate.

Le substrat utilisé dans la présente invention peut être en combinaison avec d'autres substrats, tels qu'un substrat d'osidase, estérase et notamment phosphatase ou phospholipase, peptidase et notamment coagulase. En particulier, le milieu peut comprendre deux substrats d'alpha glucosidase.

Les substrats de l'invention sont utilisables dans une large gamme de pH, notamment entre pH 5,5 et 10, préférentiellement entre 6,5 et 9.

Au sens de la présente invention, un antibiotique qui appartient à la famille des céphalosporines est un antibiotique préférentiellement choisi parmi
o Une céphalosporine de première génération, telle que: Cefalexine, Cefaloridine, Cefalotine, Cefazoline, Cefadroxil, Cefazedone, Cefatrizine, Cefapirine, Cefradine, Cefacetrile, Cefrodaxine, Ceftezole
o Une Céphalosporine de deuxième génération, telle que : Cefoxitine, Cefuroxime, Cefamandole, Cefaclor, Cefotetan, Cefonicide, Cefotiam, Loracarbef, Cefinetazole, Cefprozil, Ceforanide, Cefminox
o Une céphalosporines de troisième génération, telle que : Cefotaxime, Ceftazidime, Cefsulodine, Ceftriaxone, Cefmenoxime, Latamoxef, Ceftizoxime, Cefixime, Cefodizime, Cefetamet, Cefpiramide, Cefoperazone, Cefpodoxime, Ceftibuten, Cefdinir, Cefditoren, Ceftriaxone, Cefoperazone, Cefbuperazone, Cefdinir
o Une céphalosporine de quatrième génération, telle que Cefepime, Cefpirome

Dans le cadre de la présente invention, l'antibiotique qui appartient à la famille des céphalosporines est préférentiellement le Cefminox ou le Cefdinir.
Au sens de la présente invention, un antibiotique qui appartient à la famille des aminosides est un antibiotique préférentiellement choisi parmi Amikacine, Gentamicine, Isepamicine, Kanamycine, Netilmicine, Streptomycine, Tobramycine.
Dans le cadre de la présente invention, l'antibiotique qui appartient à la famille des aminosides est préférentiellement l'Amikacine ou la Kanamycine.
Par échantillon biologique, on entend un échantillon clinique, issu d'un prélèvement d'aspiration bronchique, trachéale ou pulmonaire, de liquide pleural, d'un lavage broncho-alvéolaire, d'expectorations, du sang ou d'une biopsie pulmonaire, de liquide articulaire ou péricardique ; liquide biologique ou un échantillon alimentaire, issu de tout type d'aliment, ou un prélèvement de surface. Cet échantillon peut être ainsi liquide ou solide et on peut citer d'une manière non limitative, un échantillon clinique de sang, de plasma, d'urines, de fécès, de prélèvements de nez, de périnée, de gorges, de peaux, de plaies, de liquide céphalo-rachidien, un échantillon alimentaire.
Par échantillon, on entend donc le prélèvement en lui même (écouvillon, selles, aliments, ...) aussi bien que des colonies de microorganismes issus dudit prélèvement (par exemple après isolement sur un milieu de culture gélifié, ou dans un bouillon d'enrichissement ensemencé avec ledit prélèvement).

A ce titre, l'invention concerne un milieu réactionnel pour la détection et/ou l'identification de bactéries *Staphylocaccus aureus* résistantes à la Méticilline (MRSA) comprenant un substrat chromogène, un premier antibiotique qui appartient à la famille des céphalosporines et un deuxième antibiotique qui appartient à la famille des aminosides. Conformément à l'invention revendiquée, ledit deuxième antibiotique est la Kanamycine ou l'Amikacine. La concentration en Kanamycine est comprise entre 0,2 et 1,5 mg/l, préférentiellement entre 0,5 et 1,25 mg/l. La concentration en Amikacine est comprise entre 0,5 et 5 mg/l, préférentiellement entre 1 et 2 mg/l.

Selon un mode préféré de réalisation de l'invention, la combinaison de deux antibiotiques comprend la Cefminox et la Kanamycine.
Selon un autre mode préféré de réalisation de l'invention, la combinaison de deux antibiotiques comprend la Cefminox et l'Amikacine.
Selon un autre mode préféré de réalisation de l'invention, la combinaison de deux antibiotiques comprend le Cefdinir et la Kanamycine.
Selon un autre mode préféré de réalisation de l'invention, la combinaison de deux antibiotiques comprend le Cefdinir et l'Amikacine.

Selon un mode préféré de réalisation de l'invention, ledit substrat chromogène permet la détection d'une activité osidase, estérase ou peptidase
Selon un mode préféré de réalisation de l'invention, ladite activité osidase est une activité alpha-glucosidase.
Ledit substrat d'une activité alpha-glucosidase est préférentiellement un indoxyl-alpha-glucoside. Préférentiellement, le substrat utilisé est le 5-Bromo-4-chloro-3-indoxyl-alpha-glucoside (X-alpha-glucoside) ou le 5-Bromo-4-chloro-3-indoxyl-N-méthyl-alpha-glucoside (GreenA-alpha-glucoside). Préférentiellement, ce substrat est présent dans le milieu à une concentration comprise entre 0,01 et 0,2 g/l, préférentiellement entre 0,03 et 0,15 g/l. En effet, à cette concentration de substrat, on obtient un meilleur contraste de coloration.
Selon un mode préféré de réalisation de l'invention, ladite activité estérase est une activité phosphatase. Préférentiellement, le substrat utilisé est le 6-Chloro-3-indoxyl-phosphate (Rose-Phosphate). Préférentiellement, ce substrat est présent dans le milieu à une concentration comprise entre 0,05 et 0,5 g/l, préférentiellement entre 0,1 et 0,4 g/l. En effet, à cette concentration de substrat, on obtient un meilleur contraste de coloration.
Selon un mode particulier de réalisation de l'invention, ledit milieu réactionnel comprend, en outre, un deuxième substrat enzymatique. Préférentiellement, ledit deuxième substrat est un substrat d'alpha-glucosidase. Préférentiellement, ledit premier substrat est un substrat d'alpha-glucosidase, préférentiellement le X-alpha-glucoside (5-Bromo-4-chloro-3-indoxyl-alpha-glucoside) et ledit deuxième substrat est un substrat d'alpha-glucosidase, préférentiellement le GreenA-alpha-glucoside (5-Bromo-4-chloro-3-indoxyl-N-méthyl-alpha-glucoside).
Selon un mode préféré de réalisation de l'invention, le milieu comprend, en outre, au moins un inhibiteur qui privilégie la croissance des bactéries *Staphylococcus aureus,* tel que Chlorure de lithium (LiCI), Azide de sodium (NaN3), Colistine, Amphotéricine, Aztréonam,

Polymixines, Chlorure de sodium(NaCl) et Déféroxamine.
Selon un mode préféré de réalisation de l'invention, le milieu comprend, en outre, un mélange d'inhibiteurs, comprenant quatre inhibiteurs, qui privilégie la croissance des bactéries *Staphylococcus aureus,* qui sont LiCl, composé vibriostatique O/129, Aztréonam, et. Amphotéricine.
L'invention concerne également l'utilisation *in vitro* d'un milieu réactionnel tel que défini ci avant pour isoler et identifier des bactéries *Staphylococcus aureus* résistantes à la Méticilline (MRSA).
L'invention concerne également un procédé de détection et/ou d'identification de bactéries *Staphylococcus aureus* résistantes à la Méticilline (MRSA) dans un échantillon biologique selon lequel
a) on ensemence l'échantillon biologique susceptible de contenir des bactéries *Staphylococcus aureus* résistantes à la Méticilline MRSA sur un milieu réactionnel tel que défini ci avant
b) on incube
c) on identifie les colonies comme étant des colonies de MRSA.
L'incubation est préférentiellement réalisée à une température comprise entre 30°C et 42°C.
Les MRSA sont préférentiellement détectées par une ou deux activités α-glucosidase ou une activité phosphatase qui permet d'obtenir des colonies colorées ou fluorescentes. Les autres espèces de *Staphylococcus* apparaissent incolores ou d'une couleur ou fluorescence différente de celle des colonies de *S. aureus.*
L'étape a) peut être précédée d'une étape de pre-enrichissement en milieu sélectif ou non sélectif.

L'invention concerne également un milieu réactionnel pour la détection et/ou l'identification de bactéries *Staphylococcus aureus* résistantes à la Méticilline (MRSA) comprenant un substrat d'une activité enzymatique ou métabolique, un premier antibiotique choisi parmi le Cefdinir et le Cefminox, et un deuxième antibiotique qui appartient à la famille des aminosides.
Préférentiellement, la concentration en Cefminox est comprise entre 10 et 35 mg/l, préférentiellement entre 12 et 28 mg/l. Préférentiellement, la concentration en Cefdinir est comprise entre 0,1 et 1 mg/l, préférentiellement entre 0,2 et 0,6 mg/l.
Selon un mode préféré de réalisation de l'invention, ledit deuxième antibiotique est la Kanamycine ou l'Amikacine.

Préférentiellement, la concentration en Kanamycine est comprise entre 0,2 et 1,5 mg/l, préférentiellement entre 0,5 et 1,25 mg/l. Préférentiellement, la concentration en Amikacine est comprise entre 0,5 et 5 mg/l, préférentiellement entre 1 et 2 mg/l)

Selon un mode préféré de réalisation de l'invention, la combinaison de deux antibiotiques comprend la Cefminox et la Kanamycine.

Selon un autre mode préféré de réalisation de l'invention, la combinaison de deux antibiotiques comprend la Cefminox et l'Amikacine.

Selon un autre mode préféré de réalisation de l'invention, la combinaison de deux antibiotiques comprend le Cefdinir et la Kanamycine.

Selon un autre mode préféré de réalisation de l'invention, la combinaison de deux antibiotiques comprend le Cefdinir et l'Amikacine.

Selon un mode préféré de réalisation de l'invention, ledit substrat d'une activité enzymatique ou métabolique permet la détection d'une activité osidase, estérase ou peptidase Selon un mode préféré de réalisation de l'invention, ladite activité osidase est une activité alpha-glucosidase.

Ledit substrat d'une activité alpha-glucosidase est préférentiellement un indoxyl-alpha-glucoside. Préférentiellement, le substrat utilisé est le 5-Bromo-4-chloro-3-indoxyl-alpha-glucoside (X-alpha-glucoside) ou le 5-Bromo-4-chloro-3-indoxyl-N-méthyl-alpha-glucoside (GreenA-alpha-glucoside). Préférentiellement, ce substrat est présent dans le milieu à une concentration comprise entre 0,01 et 0,2 g/l, préférentiellement entre 0,03 et 0,15 g/l. En effet, à cette concentration de substrat, on obtient un meilleur contraste de coloration.

Selon un mode préféré de réalisation de l'invention, ladite activité estérase est une activité phosphatase. Préférentiellement, le substrat utilisé est le 6-Chloro-3-indoxyl-phosphate (Rose-Phosphate). Préférentiellement, ce substrat est présent dans le milieu à une concentration comprise entre 0,05 et 0,5 g/l, préférentiellement entre 0,1 et 0,4 g/l. En effet, à cette concentration de substrat, on obtient un meilleur contraste de coloration.

Selon un mode particulier de réalisation de l'invention, ledit milieu réactionnel comprend, en outre, un deuxième substrat enzymatique. Préférentiellement, ledit deuxième substrat est un substrat d'alpha-glucosidase. Préférentiellement, ledit premier substrat est un substrat d'alpha-glucosidase, préférentiellement le X alpha glucoside (5-Bromo-4-chloro-3-indoxyl-alpha-glucoside) et ledit deuxième substrat est un substrat d'alpha-glucosidase, préférentiellement le GreenA-alpha-glucoside (5-Bromo-4-chloro-3-indoxyl-N-méthyl-alpha-glucoside).

Selon un mode préféré de réalisation de l'invention, le milieu comprend, en outre, au moins un inhibiteur qui privilégie la croissance des bactéries *Staphylococcus aureus,* tel que Chlorure de lithium (LiCI), Azide de sodium (NaN3), Colistine, Amphotéricine, Aztréonam, Polymixines, Chlorure de sodium(NaCl) et Déféroxamine.
Selon un mode préféré de réalisation de l'invention, le milieu comprend, en outre, un mélange d'inhibiteurs, comprenant quatre inhibiteurs, qui privilégie la croissance des bactéries *Staphylococcus aureus,* qui sont LiCI, composé vibriostatique O/129, Aztréonam, et. Amphotéricine.
L'invention concerne également l'utilisation *in vitro* d'un milieu réactionnel tel que défini ci avant pour isoler et identifier des bactéries *Staphylococcus aureus* résistantes à la Méticilline (MRSA).
L'invention concerne également un procédé de détection et/ou d'identification de bactéries *Staphylococcus aureus* résistantes à la Méticilline (MRSA) dans un échantillon biologique selon lequel
a) on ensemence l'échantillon biologique susceptible de contenir des bactéries *Staphylococcus aureus* résistantes à la méticilline MRSA sur un milieu réactionnel tel que défini ci avant
b) on incube
c) on identifie les colonies comme étant des colonies de MRSA.
L'incubation est préférentiellement réalisée à une température comprise entre 30°C et 42°C.
Les MRSA sont préférentiellement détectées par une ou deux activités alpha-glucosidase ou une activité phosphatase qui permet d'obtenir des colonies colorées ou fluorescentes. Les autres espèces de *Staphylococcus* apparaissent incolores ou d'une couleur ou fluorescence différente de celle des colonies de *S. aureus.*
L'étape a) peut être précédée d'une étape de pré-enrichissement en milieu sélectif ou non sélectif.

Les exemples suivants sont donnés à titre illustratif et n'ont aucun caractère limitatif. Ils permettront de mieux comprendre l'invention.

### EXEMPLE 1 - MILIEU MRSA COMPRENANT UNE COMBINAISON D'ANTIOBIOTIQUES CEFMINOX - KANAMYCINE ET DIFFERENTS SUBSTRATS CHROMOGENES

### 1. Préparation du milieu selon l'invention

Les milieux testés dans les expériences étaient les suivants:
**Milieu T** : milieu témoin chromID™ MRSA (réf. 43 451)
**Milieu A :** milieu T, la céfoxitine étant substituée par une combinaison d'antibiotiques Cefminox (Daewoo Chemical, A8060902; 16 mg/l) / kanamycine (Sigma, réf K4000; 1 mg/l), le substrat chromogène étant le Rose-phosphate (Biosynth, réf. C-5100 ; 0,25 g/l)
**Milieu B** : milieu T, la céfoxitine étant substituée par une combinaison d'antibiotiques Cefminox (16 mg/l) / kanamycine (0,75 mg/l), le substrat chromogène étant le X-alpha-glucoside (Biosynth, B7230; 0,045 g/l) et le Green A-alpha-glucoside (Inalco, réf. 1758-0730)
**Milieu C :** milieu T, la céfoxitine étant substituée par une combinaison d'antibiotiques Cefminox (16 mg/l) / kanamycine (0,75 mg/l), les substrats chromogènes étant le X-alpha-glucoside (Biosynth, B7230; 0,045 g/l) et le Green A-alpha-glucoside (Inalco, réf. 1758-0730, 0.08 g/l)

### 2. Ensemencement et lecture des milieux

Différentes souches de bactéries *S aureus,* résistantes (MRSA) ou non résistantes à la méticilline (MSSA) et des souches de staphylocoques à coagulase négative (SCN), toutes issues de la collection de la Demanderesse, ont été mises en suspension dans de l'eau physiologique, puis ensemencées sur le milieu, selon la technique des quatre quadrants. Les boîtes ont été incubées à 37 °C pendant 24 heures. Les colonies formées ont été examinées visuellement après 18h et 24 heures d'incubation.

### 3. Résultats :

Les résultats obtenus sont présentés dans le tableau 1.

| **Milieu** | **MRSA détectées / MRSA testées** | **MSSA + SCN détectées / MSSA + SCN testées** |
|---|---|---|
| **T** | 18/20 | 1/15 |
| **A** | 20/20 | 5/18 |
| **B** | 19/20 | 4/15 |
| **C** | 29/35 | 4/24 |

Ces résultats montrent une excellente sensibilité des milieux selon l'invention. En particulier, le milieu A permettait de détecter l'ensemble des MRSA.

### EXEMPLE 2 - MILIEU MRSA COMPRENANT DE LA CEFMINOX ET DE LA KANAMYCINE A DIFFERENTES CONCENTRATIONS

### 1. Préparation du milieu selon l'invention

Les milieux testés dans les expériences étaient les suivants:
**Milieu T :** milieu témoin chromID™ MRSA (réf. 43 451)
**Milieu D :** milieu T, la céfoxitine étant substituée par une combinaison d'antibiotiques cefminox (antibiotique identique à celui utilisé dans l'exemple 1) / kanamycine (antibiotique identique à celui utilisé dans l'exemple 1), à différentes concentrations, les substrats chromogènes étant le X-alpha-glucoside (substrat identique à celui utilisé dans l'exemple 1 ; 0,045 g/l) et le Green A-alpha-glucoside (substrat identique à celui utilisé dans l'exemple 1 ; 0,08 g/l)

| | [cefminox] en mg/l | [kanamycine] en mg/l |
|---|---|---|
| D1 | 16 | 0,75 |
| D2 | 16 | 1 |
| D3 | 14 | 0,75 |
| D4 | 14 | 1 |

### 2. Ensemencement et lecture des milieux

Cette étape a été réalisée comme dans l'exemple 1

### 3. Résultats :

Les résultats obtenus sont présentés dans le tableau 2 ci dessous

| **Milieu** | **MRSA détectées / MRSA testées** | **MSSA + SCN détectées / MSSA + SCN testées** |
|---|---|---|
| **T** | 18/20 | 1/15 |
| **D1** | 19/20 | 1/18 |
| **D2** | 15/18 | 0/18 |
| **D3** | 16/18 | 1/18 |
| **D4** | 16/18 | 1/18 |

Ces résultats montrent qu'une concentration en Cefminox de 16 mg/l et une concentration de Kanamycine de 0,75 mg/l permettait d'obtenir une excellente spécificité et sensibilité.

### EXEMPLE 3 - MILIEU MRSA COMPRENANT UNE COMBINAISON D'ANTIOBIOTIQUES CEFMINOX - AMIKACINE ET DIFFERENTS SUBSTRATS CHROMOGENES

### 1. Préparation du milieu selon l'invention

Les milieux testés dans les expériences étaient les suivants:
**Milieu T :** milieu témoin chromID™ MRSA (réf. 43 451)
**Milieu E :** milieu T, la céfoxitine étant substituée par une combinaison d'antibiotiques Cefminox (antibiotique identique à celui utilisé dans l'exemple 1; 16 mg/l) / amikacyne (Sigma, réf 2324; 2 mg/l), le substrat chromogène étant le Rose-Phosphate (Biosynth, C-5100; 0,25 g/l)
**Milieu F :** milieu T, la céfoxitine étant substituée par une combinaison d'antibiotiques Cefminox (16 mg/l) / amikacyne (2,5 mg/l), les substrats chromogènes étant le X-alpha-glucoside (substrat identique à celui utilisé dans l'exemple 1; 0,045 g/l) et le Green A-alpha-glucoside (substrat identique à celui utilisé dans l'exemple 1; 0,08 g/l)

### 2. Ensemencement et lecture des milieux

Cette étape a été réalisée comme dans l'exemple 1.

### 3. Résultats :

Les résultats obtenus sont présentés dans le tableau 3 ci dessus

| **Milieu** | **MRSA détectées / MRSA testées** | **MSSA + SCN détectées / MSSA + SCN testées** |
|---|---|---|
| **T** | 18/20 | 1/15 |
| **E** | 20/20 | 3/15 |
| **F** | 19/20 | 6/24 |

Ces résultats montrent que la combinaison d'antibiotiques selon l'invention permettait d'obtenir une excellente sensibilité, et ce quel que soit le substrat utilisé.

### EXEMPLE 4 - MILIEU MRSA COMPRENANT DE LA CEFDINIR ET DE L'AMIKACINE

### 1. Préparation du milieu selon l'invention

Les milieux testés dans les expériences étaient les suivants:
**Milieu T :** milieu témoin chromID™ MRSA (réf. 43 451)
**Milieu G :** milieu T, la céfoxitine étant substituée par une combinaison d'antibiotiques cefdinir (CDR 0806007) / amikacine (antibiotique identique à celui utilisé dans l'exemple 3) à différentes concentrations, le substrat chromogène étant le Rose-phosphate (substrat identique à celui utilisé dans l'exemple 3; 0,25 g/l)

| | [cefdinir] en mg/l | [amikacine] en mg/l |
|---|---|---|
| G1 | 0,3 | 2 |
| G2 | 0,2 | 2 |

**Milieu H :** milieu T, la céfoxitine étant substituée par une combinaison d'antibiotiques cefdinir / amikacine , à différentes concentrations, le substrat chromogène étant le X-alpha-glucoside (substrat identique à celui utilisé dans l'exemple 1; 0,045 g/l) et le Green A-alpha-Glu (substrat identique à celui utilisé dans l'exemple 1, 0.08 g/l)

| | [cefdinir] en mg/l | [amikacine] en mg/l |
|---|---|---|
| H1 | 0,3 | 2 |
| H2 | 0,2 | 2 |

### 2. Ensemencement et lecture des milieux

Cette étape a été réalisée comme dans l'exemple 1

### 3. Résultats :

Les résultats obtenus sont présentés dans le tableau 4 ci dessous.

| **Milieu** | **MRSA détectées / MRSA testées** | **MSSA + SCN détectées / MSSA + SCN testées** |
|---|---|---|
| T | 18/20 | 1/15 |
| G1 | 20/20 | 0/15 |
| G2 | 20/20 | 1/15 |
| H1 | 18/20 | 1/15 |
| H2 | 19/20 | 2/15 |

Ces résultats montrent que la combinaison d'antibiotiques Cefdinir / Amikacine selon l'invention permettait d'obtenir une excellente sensibilité et spécificité, et ce, quelle que soit la concentration utilisée.

### EXEMPLE 5 - ANALYSES DE PRELEVELEMENTS CLINIQUE SUR DES MILIEUX MRSA SELON L'INVENTION

### 1. Préparation du milieu selon l'invention

Les milieux testés dans les expériences étaient les suivants:
Milieu T : milieu témoin chromID™ MRSA (réf. 43 451)
Lot 1 : Green A- alpha -Glu 0.08 g/l + X-alpha -Glu 0.045 g/l, cefminox 16 mg/l + kanamycine 0.75 mg/l
Lot 2 : Green A- alpha -Glu 0.08 g/l + X-alpha-Glu 0.045 g/l, cefdinir 0.3 mg/l + amikacine 2 mg/l
Lot 3 : Rose-Phosphate 0.25 g/l, Cefminox 16 mg/l + amikacine 2 mg/l
Lot 4 : Rose-Phosphate 0.25 g/l, cefdinir 0.3 mg/l + amikacine 2 mg/l

Les substrats et antibiotiques utilisés étaient les mêmes que ceux utilisés dans les exemples précédents.

### 2. Ensemencement et lecture des milieux

Deux études ont été réalisées en parallèle à partir de différents prélèvements cliniques (nez, gorge, périnée, plaie), qui ont été mis en suspension dans de l'eau physiologique, et ensemencés pour donner des colonies isolées sur le milieu, selon la technique des quatre quadrants. Les boîtes ont été incubées à 37 °C pendant 24 heures. Les colonies formées ont été examinées visuellement après 18h et 24 heures d'incubation.

### 3. Résultats :

Les résultats obtenus sont présentés dans les tableaux ci dessous.

**Première étude:**

| **Paramètres** | **temps** | **Milieu T** | **Lot 1** | **Lot 2** | **Lot 3** | **Lot 4** |
|---|---|---|---|---|---|---|
| **Sensibilité de détection** | 18 h | 15/19 | 17/19 | 16/19 | 18/19 | 18/19 |
| | 24 h | 18/19 | 19/19 | 19/19 | 19/19 | 19/19 |
| **Spécificité** | 24 h | 97% | 86% | 88% | 82% | 80% |

**Deuxième étude:**

| **Paramètres** | **temps** | **Milieu T** | **Lot 1** | **Lot 2** | **Lot 3** | **Lot 4** |
|---|---|---|---|---|---|---|
| **Sensibilité de détection** | 18 h | 48/60 | 49/60 | 31/60 | 47/60 | 54/60 |
| | 24 h | 56/60 | 54/60 | 42/60 | 48/60 | 57/60 |
| **Spécificité** | 24 h | 95% | 93% | 89% | 99% | 98% |

Ces résultats montrent que les combinaisons d'antibiotiques selon l'invention présentaient une excellente sensibilité de détection, et ceci dès 18h.

## Revendications

1. Milieu réactionnel pour la détection et/ou l'identification de bactéries *Staphylococcus aureus* résistantes à la Méticilline (MRSA) comprenant un substrat chromogénique, un premier antibiotique qui appartient à la famille des céphalosporines et un deuxième antibiotique qui appartient à la famille des aminosides et qui est choisi entre la Kanamycine à une concentration comprise entre 0,2 et 1,5 m/l, et l'Amikacine à une concentration comprise entre 0,5 et 5 mg/L.

2. Milieu réactionnel selon la revendication 1 selon lequel ledit premier antibiotique est la Cefminox ou le Cefdinir.

3. Milieu réactionnel selon la revendication 1 ou 2 selon lequel ledit substrat chromogène permet la détection d'une activité osidase, estérase ou peptidase

4. Milieu réactionnel selon la revendication 3, selon lequel ladite activité osidase est une activité alpha-glucosidase.

5. Milieu réactionnel selon la revendication 3, selon lequel ladite activité estérase est une activité phosphatase.

6. Utilisation in vitro d'un milieu réactionnel selon l'une quelconque des revendications 1 à 5 pour isoler et identifier des bactéries Staphylococcus aureus résistantes à la Méticilline (MRSA).

7. Procédé de détection et/ou d'identification de bactéries Staphylococcus aureus résistantes à la Méticilline (MRSA) dans un échantillon biologique selon lequel
a) on ensemence l'échantillon biologique susceptible de contenir des bactéries *Staphylococcus aureus* résistantes à la méticilline MRSA sur un milieu réactionnel selon l'une quelconque des revendications 1 à 5
b) on incube
c) on identifie les colonies comme étant des colonies de MRSA.

## Patentansprüche

1. Reaktionsmedium für den Nachweis und/oder die Identifikation von Methicillin-resistenten *Staphylococcus* aureus(MRSA)-Bakterien, umfassend ein chromogenes Substrat, ein erstes Antibiotikum, das zur Familie der Cephalosporine gehört, und ein zweites Antibiotikum, das zur Familie der Aminoside gehört und das aus Kanamycin in einer Konzentration zwischen 0,2 und 1,5 mg/l und Amikacin in einer Konzentration zwischen 0,5 und 5 mg/l ausgewählt ist.

2. Reaktionsmedium nach Anspruch 1, wobei es sich bei dem ersten Antibiotikum um Cefminox oder Cefdinir handelt.

3. Reaktionsmedium nach Anspruch 1 oder 2, wobei das chromogene Substrat den Nachweis einer Osidase-, Esterase- oder Peptidaseaktivität gestattet.

4. Reaktionsmedium nach Anspruch 3, wobei es sich bei der Osidaseaktivität um eine alpha-Glucosidaseaktivität handelt.

5. Reaktionsmedium nach Anspruch 3, wobei es sich bei der Esteraseaktivität um eine Phosphataseaktivität handelt.

6. In-vitro-Verwendung eines Reaktionsmediums nach einem der Ansprüche 1 bis 5 zum Isolieren und Identifizieren von Methicillin-resistenten *Staphylococcus aureus*(MRSA)-Bakterien.

7. Verfahren für den Nachweis und/oder die Identifikation von Methicillin-resistenten *Staphylococcus aureus*(MRSA)-Bakterien in einer biologischen Probe, wobei
a) man eine biologische Probe, die Methicillinresistente *Staphylococcus aureus*(MRSA)-Bakterien enthalten kann, auf ein Reaktionsmedium nach einem der Ansprüche 1 bis 5 impft,
b) man inkubiert,
c) man die Kolonien als MRSA-Kolonien identifiziert.

## Claims

1. Reaction medium for detecting and/or identifying methicillin-resistant *Staphylococcus aureus* (MRSA) bacteria, comprising a chromogenic substrate, a first antibiotic which belongs to the cephalosporin family and a second antibiotic which belongs to the aminoglycoside family and is chosen between kanamycin a concentration between 0.2 and 1.5 mg/l and amakacin at a concentration between 0.5 and 5 mg/l.

2. Reaction medium according to claim 1, wherein said first antibiotic is cefminox or cefdinir.

3. Reaction medium according to claim 1 or 2, wherein said chromogenic substrate allows the detection of an osidase, esterase or peptidase activity.

4. Reaction medium according to claim 3, according to which said osidase activity is an alpha-glucosidase activity.

5. Reaction medium according to claim 3, in which said esterase activity is a phosphatase activity.

6. *In vitro* use of a reaction medium according to any one of claims 1 to 5, for isolating and identifying methicillin-resistant *Staphylococcus aureus* (MRSA) bacteria.

7. Method for detecting and/or identifying methicillin-resistant *Staphylococcus aureus* (MRSA) bacteria in a biological sample, comprising:
a) inoculating the biological sample that may contain methicillin-resistant *Staphylococcus aureus* (MRSA) bacteria on a reaction medium according to any one of claims 1 to 5;
b) incubating;
c) identifying the colonies as being MRSA colonies.
